(19) Europäisches Patentamt
European Patent Office
Office européen des brevets

(11) Publication number: **0 279 948**
**A1**

# (12) EUROPEAN PATENT APPLICATION

(21) Application number: **87119241.5**

(22) Date of filing: **28.12.87**

(51) Int. Cl.4: **C07D 311/92** , C07D 493/10 , C07D 497/10 , A61K 31/35

Claims for the following Contracting States: ES + GR.

(30) Priority: **29.12.86 US 947070**

(43) Date of publication of application: **31.08.88 Bulletin 88/35**

(84) Designated Contracting States: **AT BE CH DE ES FR GB GR IT LI LU NL SE**

(71) Applicant: **HOECHST-ROUSSEL PHARMACEUTICALS INCORPORATED**
**Route 202-206 North**
**Somerville New Jersey 08876(US)**

(72) Inventor: **Kosley, Raymond Walter, Jr.**
**659 Cedarbrook Road**
**Bridgewater, N.J. 08807(US)**
Inventor: **Cherill, Robert Joseph**
**80 Koasuth Street**
**Somerset, N.J. 08873(US)**

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80(DE)**

(54) **Novel carbamoyloxylabdanes, intermediates and a process for the preparation thereof and their use as medicaments.**

(57) The present invention relates to carbamoyloxylabdanes, intermediates and a process for the preparation thereof. The compounds of the invention exhibit intraocular pressure activity and can, therefore, be used as medicaments.

EP 0 279 948 A1

# Novel carbamoyloxylabdanes, intermediates and a process for the preparation thereof and their use as medicaments

The present invention relates to carbamoyloxylabdanes of the formula 1

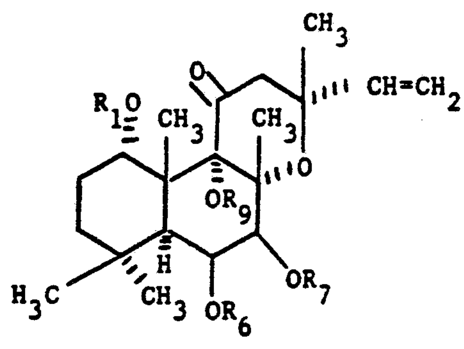

1

wherein:

(a) $R_1$ is hydrogen or a group of the formula $R_2R_3N\overset{O}{\overset{\|}{C}}$ wherein $R_2$ and $R_3$ are independently hydrogen or loweralkyl of 1 to 6 carbon atoms; or $R_2$ and $R_3$ taken together with the nitrogen atom to which they are attached form a group of the formula

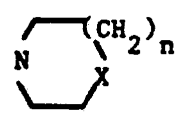

wherein X is O, S or a group of the formula $CHR_4$ wherein $R_4$ is hydrogen, loweralkyl, or a group of the formula $OR_5$ wherein $R_5$ is hydrogen, loweralkyl or a group of the formula $\overset{O}{\overset{\|}{C}}R_{10}$ wherein $R_{10}$ is loweralkyl and n is 0 or 1;

(b) $R_9$ is hydrogen;

(c) $R_1$ and $R_9$ taken together form a group of the formula CO, a group of the formula SO or a group of the formula $CHNR_{11}R_{12}$ wherein $R_{11}$ and $R_{12}$ are each independently loweralkyl of 1 to 6 carbon atoms; and $R_{11}$ and $R_{12}$ taken together with the nitrogen atom to which they are attached form a group of the formula

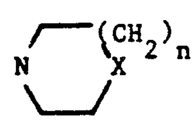

wherein X and n are as above;

(d) $R_6$ and $R_7$ are idenpendently hydrogen or a group of the formula $R_{13}R_{14}N\overset{O}{\overset{\|}{C}}$ wherein $R_{13}$ is hydrogen, loweralkyl of 1 to 6 carbon atoms, a group of the formula $R_{15}O(CH_2)_p$wherein $R_{15}$ is hydrogen or loweralkyl of 1 to 6 carbon atoms and p is 0, 2 or 3 or a group of the formula $HOCH_2CH(OH)CH_2$; $R_{14}$ is hydrogen, loweralkyl of 1 to 6 carbon atoms, a group of the formula $R_{16}O$-$(CH_2)_q$ wherein $R_{16}$ is hydrogen and q is 2 or 3; or $R_{13}$ and $R_{14}$ taken together with the nitrogen atom to which they are attached form a group of the formula

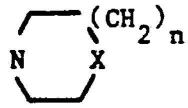

wherein X and n are as above, with the provisos:

(e) that $R_{1}$, $R_{6}$ and $R_{7}$ are not simultaneously hydrogen,

(f) that when $R_{1}$ and $R_{6}$ are hydrogen, $R_{13}$ and $R_{14}$ are not simultaneously loweralkyl of 1 to 6 carbon atoms, and

(g) that when $R_{1}$ and $R_{9}$ taken together form a group of the formula CO, SO or $CHNR_{11}R_{12}$, $R_{6}$ and $R_{7}$ are not simultaneously hydrogen; the optical or geometric isomers thereof, which are useful for reducing intraocular pressure, alone or in combination with inert adjuvants.

Subgeneric to the carbamoyloxylabdanes of the present invention are compounds of formula $\underline{1}$ wherein:

(a) $R_{1}$ is hydrogen and $R_{7}$ is a group of the formula

$R_{13}R_{14}N\overset{O}{\overset{||}{C}}$ wherein $R_{13}$ and $R_{14}$ are as above;

(b) $R_{1}$ is hydrogen and $R_{6}$ is a group of the formula

$R_{13}R_{14}N\overset{O}{\overset{||}{C}}$ wherein $R_{13}$ and $R_{14}$ are as above;

(c) $R_{1}$ is a group of the formula $R_{2}R_{3}N\overset{O}{\overset{||}{C}}$ wherein $R_{2}$ and $R_{3}$ are as above and $R_{6}$ and $R_{7}$ are hydrogen;

(d) $R_{1}$ and $R_{9}$ taken together form a group of the formula $CHNR_{11}R_{12}$ wherein $R_{11}$ and $R_{12}$ are as above and $R_{7}$ is

a group of the formula $R_{13}R_{14}N\overset{O}{\overset{||}{C}}$ wherein $R_{13}$ and $R_{14}$ are as above; and

(e) $R_{1}$ is hydrogen, $R_{6}$ is hydrogen, $R_{13}$ is hydrogen and $R_{14}$ is loweralkyl of 1 to 6 carbon atoms.

As used through the specification and appended claims, the term "alkyl" refers to a straight or branched chain hydrocarbon radical containing no unsaturation and having 1 to 8 carbon atoms such as methyl, ethyl, 1-propyl, 2-propyl, 1-butyl, 1-pentyl, 2-pentyl, 3-hexyl, 4-heptyl, 2-octyl, and the like; the term "alkanol" refers to a compound formed by a combination of an alkyl group and a hydroxy radical. Examples of alkanols are methanol, ethanol, 1-and 2-propanol, 1, 2-dimethylethanol, hexanol, octanol and the like. The term "alkanoic acid" refers to a compound formed by combination of a carboxyl group with a hydrogen atom or alkyl group. Examples of alkanoic acids are formic acid, acetic acid, propanoic acid, 2, 2-dimethylacetic acid, hexanoic acid, octanoic acid, and the like; the term "halogen" refers to a member of the family consisting of fluorine, chlorine, bromine or iodine. The term "lower" as applied to any of the aforementioned groups refers to a group having a carbon skeleton containing up to and including 6 carbon atoms.

In the formula presented herein the various substituents are illustrated as joined to the labdane nucleus by one of two notations: a solid line (———) indicating a substituent which is in the $\beta$-orientation (i.e., above the plane of the molecule) and a broken line (---) indicating a substituent which is in the $\alpha$-orientation (i.e, below the plan of the molecule). The formulas have all been drawn to show the compounds in their absolute sterochemical configuration. Inasmuch as the starting materials having a labdane nucleus are naturally occurring or are derived from naturally occurring materials, they, as well as the final products, have a labdane nucleus existing in the single absolute configuration depicted herein. The processes of the present invention, however, are intended to apply as well to the synthesis of labdanes of the racemic series.

In addition to the optical centers of the labdane nucleus, the substituents thereon may also contain chiral centers contributing to the optical properties of the compounds of the present invention and providing a means for the resolution thereof by conventional methods. A wavy line ( ∼ ) connecting a group to a chiral center indicates that the stereochemistry of the center is unknown, i.e, the group may exist in any of the possible orientations. The present invention comprehends all optical isomers and racemic forms of the compounds of the present invention where such compounds have chiral centers in addition to those of the labdane nucleus.

The noval labdanes of the present invention are synthesized by the processes illustrated in Reaction Schemes A and B.

To prepare a carbamoyloxylabdane $\underline{4}$, an 8, 13-epoxy-1$\alpha$, 6$\beta$, 9$\alpha$-trihydroxylabd-14-en-11-one-1, 9-dialkylformamide acetal $\underline{2}$, the synthesis of which is described in U. S. Patent Application Serial No. 848,053, filed April 4, 1986, is carbamoylated to provide a 7$\beta$-carbamoyloxylabdane-formamide acetal $\underline{3}$ which is hydrolyzed to a 7$\beta$-carbamoyloxy-1$\alpha$, 6$\beta$, 9$\alpha$-trihydroxylabdane $\underline{4}$.

The carbamoylation is accomplished by treating a 6$\beta$, 7$\beta$-dihydroxylabdane $\underline{2}$ with 1,1'-carbonyldiimidazole $\underline{8}$

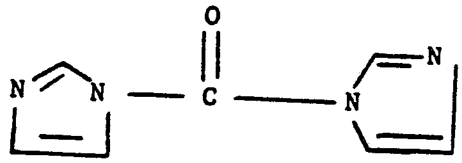

8

in an alkyl alkanoate or a halocarbon to afford an imidazolocarbonyloxylabdane of formula 9

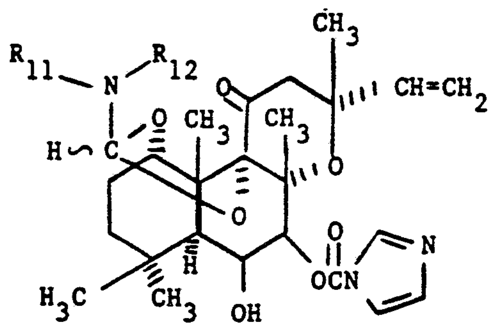

9

which, preferably without isolation, is treated with an amine of formula 10

$R_{13}R_{14}NH$ 10

wherein $R_{13}$ and $R_{14}$ are as above, neat, in an alkyl alkanoate or a mixture of a halocarbon and an alkanol to yield 3. Among alkyl alkanoates there may be mentioned methyl acetate, ethyl acetate, methyl propionate, ethyl propionate and the like. Ethyl acetate is preferred. Among halocarbons there may be mentioned dichloromethane, trichloromethane and the like. Dichloromethane is preferred. Among alkanols there may be mentioned methanol, ethanol, 2-propanol and the like. Methanol is preferred and mixtures of dichloromethane and methanol are also preferred. While the temperature at which the carbamoylation is performed is not narrowly critical, it is preferred to carry out the reaction at a temperature between about 0°C to about 50°C, most preferrably at a temperature of about 25°C.

If desired, the intermediate imidazolocarbonyloxylabdane 9 may be isolated by workup of the reaction mixture prior to the addition of amine 10 by methods well-known in the art. For example, the intermediate labdane 9 may be isolated by chromatography on a suitable column (e.g, silica gel) with an appropriate eluent such as hexane/ethyl acetate.

The hydrolysis is achieved by contacting a 7β-carbamoyloxylabdaneformamide acetal 3 with aqueous alkanol, or with an aqueous alkanoic acid in alkanol, or a mineral acid in aqueous alkanol. Included among aqueous alkanoic acids are aqueous formic acid, aqueous acetic acid, aqueous propionic acid and the like. Included among mineral acids are hydrochloric acid, hydrobromic acid, sulfuric acid, nitric acid and the like. Included among alkanols are methanol, ethanol, 2-propanol and the like. A reaction medium consisting of about 80% aqueous acetic acid and methanol or consisting of methanol is preferred. The hydrolysis proceeds readily at a temperature within the range of about 0°C to about 50°C. A hydrolysis temperature of about 25°C is preferred.

Alternatively, the carbamoylation of a 6β, 7β-dihydroxylabdaneformamide acetal 2 is effected by treating such a labdane 2 with a base, for example, an alkali metal bis(trialkylsilyl) amide of formula 11

$[(R_{17})3Si]_2NLi$ 11

wherein $R_{17}$ is loweralkyl in an organic solvent, for example, an ethereal solvent, to form an alkali metal alkoxide of 2, followed by treatment with either an isocyanate of formula 12

$R_{13}NCO$ 12

wherein $R_{13}$ is as hereinbeforedescribed or a carbamoyl halide of formula 13

$R_{13}R_{14}N\overset{\overset{O}{\|}}{C}$ Hal 13

wherein $R_{13}$ and $R_{14}$ are as hereinbeforedescribed and Hal is halogen, neat or in an ethereal solvent, to

provide a 7β-carbamoyloxylabdane 3. Examples of alkali metal bis(trialkylsilyl) amides include lithium, sodium or potassium bis(trimethylsilyl)-or bis(triethylsilyl) amides and the like. Examples of ethereal solvents are diethyl ether, 1, 2-dimethoxyethane, dioxane, tetrahydrofuran and the like. A reaction medium consisting of lithium bis(trimethyl)silylamide and tetrahydrofuran is preferred. The formation of the alkali metal alkoxide is performed at a temperature within the non-critical range of about -25C to about 50°C, preferably at a temperature of about 0°C to about 25°C. The condensation of an alkali metal alkoxide of 2 with either an isocyanate 12 or a carbamoyl halide 13 is performed at a temperature of about 0°C to about the reflux temperature of the reaction medium, preferrably at about 25°C to the reflux temperature.

To prepare a 6β-carbamoyloxylabdane of formula 5, a 7β-carbamoylabdane of formula 4 is rearranged by means of an alkali metal alkoxide in an alkanol or ethereal solvent, alone or combination, at a temperature within the range of about 0°C to about 50°C, a temperature of about 25°C being preferred. Suitable alkali metal alkoxides include lithium, sodium and potassium 2-propoxides, lithium, sodium and potassium 2, 2-dimethylethoxides and the like. Suitable alkanols include 2-propanol, 2, 2-dimethylethanol and the like. Suitable ethereal solvents include diethyl ether, 1, 2-dimethoxyethane, tetrahydrofuran, dioxane and the like. Potassium 2, 2-dimethylethoxide and a combination of 2, 2-dimethylethanol and tetrahydrofuran is the preferred rearrangement medium.

To prepare a carbamoyloxy-6β, 7β, 9α-trihydroxylabdane of formula 7, 8, 13-epoxy-1α, 6β, 7β, 9α-tetrahydroxylabd-14-en-11-one 6, the synthesis of which is described in U. S. Patent 4,134,986, issued January 16, 1979 to B. S. Bajwa, et al., is contacted with 1, 1'-carbonyldiimidazole 8 in an ethereal solvent such as tetrahydrofuran followed by an amine 10 wherein $R_{13}$ and $R_{14}$ are $R_2$ and $R_3$, respectively, under conditions substantially similar to those employed for the conversion of 6β, 7β-dihydroxylabdane-1α, 9α-formamide acetal 2 to a 7β-carbamoyloxylabdane 3, as hereinbeforedescribed.

To construct a carbamoyloxylabdane having a 1α, 9α-sulfite or carbonate function, i.e., a compound of formula 15 wherein $R_{13}$ and $R_{14}$ are as above and Y is SO or CO, an 8,13-epoxy-1α, 6β, 7β, 9α-tetrahydroxylabdane-14-en-11-one-1α, 9α-sufite or -carbonate 14, the preparation of which is described in by N. J. de Souza, et al., Medicinal Research Reviews, 3, 201 (1983), may be treated with 1, 1'-carbonyldiimidazole 8 followed by an amine of formula 10, or an alkoxide of 14 may be condensed with an isocyanate 12 or carbamoyl halide 14, all steps being performed by processes substantially similar to those describedherein for the related conversion of 7β-hydroxylabdane 2 to 7β-carbamoyloxylabdane 3.

The carbamoyloxylabdanes of the present invention are useful in the treatment of glaucoma by virtue of their ability to reduce elevated intraocular pressure in a glaucomatous subject as determined by the method described by J. Caprioli, et al., Invest. Ophthalmol. Vis. Sci., 25, 268 (1984). The results of the determination expressed as percent decrease of outflow pressure is presented in the Table.

TABLE

| COMPOUND | CONCENTRATION (%) | DECREASE IN OUTFLOW PRESSURE (%) |
|---|---|---|
| 8,13-epoxy-7β-(N-methylaminocarbonyloxy)-1α,6β,9α-trihydroxylabd-14-en-11-one | 0.25 | 59 |
| 7β-(aminocarbonyloxy)-8,13-epoxy-1α-6β,9α-trihydroxylabd-14-en-11-one | 0.50 | 41 |
| 8,13-epoxy-7β-(2-hydroxyethylaminocarbonyloxy)-1α,6β,9α-trihydroxylabd-14-en-11-one | 0.25 | 50 |
| 7β-acetoxy-8,13-epoxy-1α,6β,9α-trihydroxylabd-14-en-11-one | 1.0 | 51 |
| | 0.1 | 23 |

Intraocular pressure reduction is achieved when the present carbamoyloxylabdanes are administered to a subject requiring such treatment as an effective topical dose of a 0.01 to 3.0% solution or suspension. A particularly effective amount is about 3 drops of a 0.25% preparation per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and that they do not, to any extent, limit the scope or practice of the invention.

Compounds of the present invention include:

(a) 1α-(Aminocarboxyloxy)-8, 13-epoxy-6β, 7β, 9α-trihydroxylabd-14-en-11-one;

(b) 8, 13-Epoxy-1α-(N-methylaminocarbonyloxy)-6β, 7β, 9α-trihydroxylabd-14-en-11-one;

(c) 8, 13-Epoxy-1α-(4-morpholinocarbonyloxy)-6β, 7β, 9α-trihydroxylabd-14-en-11-one;

(d) 8, 13-Epoxy-1α-(4-thiomorpholinocarbonyloxy)-6β, 7β, 9α-trihydroxylabd-14-en-11-one;

(e) 8, 13-Epoxy-1α-(4-hydroxy-1-piperidinocarbonyloxy)-6β, 7β, 9α-trihydroxylabd-14-en-11-one;

(f) 8, 13-Epoxy-1α-(4-methoxy-1-piperidinocarbonyloxy)-6β, 7β, 9α-trihydroxylabd-14-en-11-one;

(g) 8, 13-Epoxy-1α-(4-acetoxy-1-piperidinocarbonyloxy)-6β, 7β, 9α-trihydroxylabd-14-en-1-one;

(h) 8, 13-Epoxy-7β-(4-thiomorpholinocarbonyloxy)-1α, 6β, 9α-trihydroxylabd-14-en-11-one-1,9-carbonate;

(i) 8, 13-Epoxy-7β-(4-methoxy-1-piperidinocarbonyloxy-1α, 6β, 9α-trihydroxylabd-14-en-11-one-1,9-sulfite;

(j) 8, 13-Epoxy-6β-(4-thiomorpholinocarbonyloxy)-1α, 7β, 9α-trihydroxylabd-14-en-11-one-1,9-(4-thiomorpholine) formamide acetal;

(k) 8, 13-Epoxy-7β-(4-methoxy-1-piperidinocarbonyloxy)1α, 6β, 9α-trihydroxylabd-14-en-11-one-1,9-(4-morpholino) formamide acetal; and

(l) 8, 13-Epoxy-7β-(4-acetoxy-1-piperidinocarbonyloxy)-1α, 6β, 9α-trihydroxylabd-14-en-11-one-1,9-(4-acetoxy-1-piperidine) formamide acetal.

The carbamoyloxylabdanes of the present invention are also useful in the treatment of hypertension, congestive heart failure, bronchial asthma and psoriasis.

Effective amounts of the compounds of the present invention may be administered to a subject by any one of various methods, for example, orally as in capsules or tablets, parenterally in the form of sterile solutions or suspensions, in some cases intravenously in the form of sterile solutions, or suspensions, and topically in the form of solutions, suspensions or ointments and by aerosol spray.

Effective quantities of the compounds of the invention may be administered orally, for example, with an inert diluent or with an edible carrier. They may be enclosed in gelatin capsules or compressed into tablets. For the purpose of oral therapeutic administration, the aforesaid compounds may be incorporated with excipients and used in the form of tablets, troches, capsules, elixirs, suspensions, syrups, wafers, chewing gums and the like. These preparations should contain at least 0.5% of active compound, but may be varied depending upon the particular form and may conveniently be between 4% to about 70% of the weight of the unit. The amount of active compound in such composition is such that a suitable dosage will be obtained. Preferred compositions and preparations according to the present invention are prepared so that an oral dosage unit form contains between 0.1-30 milligrams of the active compound.

The tablets, pills, capsules, troches and the like may also contain the following ingredients: a binder such as microcrystalline cellulose, gum tragacanth or gelatin an excipient such as starch or lactose, a disintegrating agent such as alginic acid, corn starch and the like; a lubricant such as magnesium stearate; a glidant such as colloidal silicon dioxide; and a sweetening agent such as sucrose or saccharin or a flavoring agent such as peppermint, methyl salicylate, or orange flavoring may be added. When the dosage unit form is a capsule, it may contain, in addition to materials of the above type, a liquid carrier such as a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes and colorings and flavours. Materials used in preparing these various compositions should be pharmaceutically pure and non-toxic in the amounts used.

For the purpose of parenteral or topical therapeutic administration, the active compounds of the invention may be incorporated into a solution, suspension, ointment or cream. These preparations should contain at least 0.01% of active compound, but may be varied between 0.1 and about 5% of the weight thereof. The amount of active compounds in such compositions is such that a suitable dosage will be obtained. Preferred compositions and preparations according to the present invention are prepared so that a parenteral or topical dosage unit contains between 0.01 to 10 milligrams of active compound.

The solutions or suspensions for topical or parenteral administration may also include the following components: A sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phophates and agents for the adjustment of tonicity such as sodium chloride or dextrose. The parenteral preparation can be enclosed in ampules or disposable syringes; the topical preparation may be enclosed in multiple dose vials or dropping bottles, made of glass or plastic.

The following Examples are for illustrative purposes only. All temperatures are given in degrees Centigrade.

EXAMPLE 1

7β-(Aminocarbonyloxy)-8,13-epoxy-1α, 6β, 9α-trihydroxylabd-14-en-11-one-1, 9-dimethylformamide acetal

A solution of 484 mg of 8, 13-epoxy-1α, 6β, 7β, 9α-tetrahydrolabd-14-en-11-one-1, 9-dimethylformamide acetal in 10 ml of ethyl acetate containing 202.5 mg of 1, 1'-carbonyldiimidazole was stirred at ambient temperature overnight. Anhydrous ammonia was bubbled into the mixture for 1 min and the mixture was stirred at ambient temperature over the weekend in a sealed vessel. The mixture was filtered and evaporated. The residue was flash chromatographed on silica gel in hexane;ethyl acetate (1:1). The appropriate fractions were combined and evaporated. The residue was recrystallized from hexane:ether to afford 148 mg (26.9%) of produce, mp 98-122°.

ANALYSIS;

Calculated for $C_{24}H_{38}N_2O_7$:

61.77%C 8.23%H 6.00%N

Found:

61.73%C 8.42%H 5.49%N

EXAMPLE 2

8, 13-Epoxy-7β-(N-methylaminocarbonyloxy)-1α, 6β, 9α-trihydroxylabd-14-en-11-one-1, 9-dimethylformamide acetal

To a solution of 100 mg of 8, 13-epoxy-1α, 6β, 7β, 9α-tetrahydroxylabd-14-en-11-one-1, 9-dimethylformamide acetal in 10 ml of tetrahydrofuran was added 23µ1 of a 1 M solution of lithium bis(trimethylsilyl) amide in tetrahydrofuran. Methyl isocyanate (14µl, 13.5 mg) was added to the mixture and the mixture was stirred at ambient temperature under nitrogen overnight. The mixture was quenched with 100µ1 of water and evaporated. The residue was flash chromatographed on silica gel in hexane:ethyl acetate (1:1). The appropriate fractions were combined and evaporated to yield 30 mg (26.4%) of product, mp 189-191°.

ANALYSIS:

Calculated for $C_{25}H_{40}N_2O_7$:

62.47%C 8.41%H 5.83%N

Found:

62.59%C 8.44%H 5.65%N

EXAMPLE 3

8, 13-Epoxy-7β-m(4-morpholinocarbonyloxy)-1α, 6β, 9α-trihydroxylabd-14-en-11-one-1, 9-dimethylformamide acetal

A solution of 500 mg of 8, 13-epoxy-1α, 6β, 7β-9α-tetrahydroxylabd-14-en-11-one-1, 9-dimethylformamide acetal in 10 ml of ethyl acetate containing 202.5 mg of 1, 1'-carbonyldiimidazole was stirred at ambient temperature under nitrogen over the weekend. Morpholine (500 ml) was added and the mixture was stirred at ambient temperature for 24 hr. The mixture was diluted with 100 ml of ethyl acetate and washed with 0.01 N hydrochloric acid until the aqueous washings remained acidic. The organic phase was washed with saturated aqueous sodium bicarbonate solution, dried over anhydrous sodium sulfate, filtered and evaporated. The residue was flash chromatographed on silica gel in hexane:ethyl acetate (1:1). The appropriate fractions were combined and evaporated to yield 180 mg (27.3%) of product, mp 75-100°.

ANALYSIS:

Calculated for $C_{28}H_{44}N_2O_5$:

62.65%C 8.28%H 5.22%N

Found:

62.60%C 8.34%H 5.07%N

EXAMPLE 4

8, 13-Epoxy-7β-(4-hydroxy-1-piperidinocarbonyloxy)-1α, 6β, 9α-trihydroxylabd-14-en-11-one-1, 9-dimethylformamide acetal

A solution of 500 mg of 8, 13-epoxy-1α, 6β, 7β, 9α-tetrahydroxylabd-14-en-11-one-1, 9-dimethylformamide acetal in 10 ml of ethyl acetate containing 202.5 mg of 1, 1'-carbonyldiimidazole was stirred at ambient temperature under nitrogen over the weekend. 4-Hydroxypiperidine (505 mg) was added and the mixture was stirred at ambient temperature for 24 hr. The mixture was diluted with 100 ml of ethyl acetate and washed with 0.01 N hydrochloric acid until the aqueous washings remained acidic. The organic layer was washed with saturated aqueous sodium bicarbonate solution, dried over anhydrous sodium sulfate, filtered and evaporated. The residue was flash chromatographed on silica gel in hexane:ethyl acetate (1:2). The appropriate fractions were combined and evaporated to yield 342.5 mg (51.9%) of product, mp 70-100°.

ANALYSIS;

Calculated for $C_{29}H_{46}N_2O_8$:

63.24%C 8.44%H 5.08%N

Found:

63.23%C 8.50%H 4.99%N

EXAMPLE 5

8, 13-Epoxy-7β-(2-hydroxyethylaminocarbonyloxy)-1α, 6β, 9α-trihydroxylabd-14-en-11-one-1, 9-dimethylformamide acetal

A solution of 1.5 g of 8, 13-epoxy-1α, 6β, 7β, 9α-tetrahydroxylabd-14-en-11-one-1, 9-dimethylformamide acetal in 30 ml of ethyl acetate containing 607.5 mg of 1,1'-carbonyldiimidazole was stirred under nitrogen at ambient temperature for 24 hr. A 10 ml-portion of this mixture was combined with a solution of 359.9 mg of 2-aminoethanol in 1 ml of ethyl acetate, and the resultant mixture was stirred for 2 days. The mixture was diluted with ethyl acetate to 100 ml and extracted several times with 0.01 N hydrochloric acid until the aqueous washings remained acidic. The ethyl acetate layer was washed with saturated aqueous sodium bicarbonate solution, dried over anhydrous sodium sulfate, filtered and evaporated. The residue was flash chromatographed on silica gel in hexane:ethyl acetate:methanol (10:10:1). The appropriate fractions were combined and evaporated and the residue was crystallized from hexane:ether to afford 201.3 mg (33.4%) of product, mp 100-110°.

ANALYSIS;

Calculated for $C_{26}H_{42}N_2O_8$:

61.15%C 8.31%H 5.48%N

Found:

60.77%C 8.34%H 5.20%N

EXAMPLE 6

7β-(2m 3-Dihydroxypropylaminocarbonyloxy)-8, 13-epoxy-1α, 6β, 9α-trihydroxylabd-14-en-11-one-1, 9-dimethylformamide acetal

A solution of 1.5 g of 8, 13-epoxy-1α, 6β, 7β, 9α-tetrahydroxylabd-14-en-11-one-1, 9-dimethylformamide acetal was dissolved in 30 ml of ethyl acetate containing 607.5 mg of 1,1'-carbonyldiimidazole and stirred under nitrogen at ambient temperature for 24 hr. A 10 ml-portion of this mixture was combined with a solution of 536.9 mg of 2, 3-dihydroxypropylamine in 1 ml of ethyl acetate and the resultant mixture was stirred for 2 days. The reaction mixture was diluted with ethyl acetate to 100 ml and extracted with 0.01 N hydrochloric acid until the aqueous washings remained acidic. The organic phase was washed with saturated aqueous sodium bicarbonate solution, dried over anhydrous sodium sulfate, filtered and evaporated. The residue was flash chromatographed on silica gel in hexane:ethyl acetate:methanol (10:10:1). The appropriate fractions were combined and evaporated. The residue was flash chromatographed on silica gel eluting first with 300 ml of hexane:ethyl acetate (2:1) then with 400 ml of hexane:ethyl acetate (1:1). The appropriate fractions were combined and evaporated. The residue was crystallized from hexane:ether to afford 189.6 mg (29.8%) of product, mp 90-100°.

ANALYSIS:
Calculated for $C_{27}H_{44}N_2O_9$:
59.97%C 8.22%H 5.18%N
Found:
59.72%C 8.20%H 4.97%N

EXAMPLE 7

7β-(N,N-Dimethylcarbamoyloxy)-8,13-epocy-1α,6β,9α-trihyroxylabd-14-en-11-one-1,9-dimethylformamide acetal

8,13-Epoxy-1α,6β,7β,9α-tetrahydroxylabd-14-en-11-one-1,9dimethylformamide acetal (500 mg) was dissolved in 50 ml of dry tetrhydrofuran under nitrogen and cooled to 0° in an ice-bath. Lithium bis-(trimethylsilyl)amide (1.4 ml of a 1M tetrahydrofuran solution)was added and the mixture was stirred at 0° for 1 hr. Dimethylcarbamoyl chloride (254 mg) was added to the mixture followed by gradual heating to reflux. The mixture was heated under reflux overnight and allowed to cool to ambient temperature. Water (100 ml) was added and the reaction mixture was evaporated. The residue was flashchromatographed on silica gel eluting first with 300 ml of hexane:ethyl acetate (2:1) then with 400 ml of hexane:ethyl acetate (1:1). The appropriate fraction were combined and evaporated. The residue was dried under vacuum to provide 231.7 mg (39.2%) product as an amorphous solid, mp 70-80°.
ANALYSIS:
Calculated for $C_{26}H_{42}N_2O_7$:
63.12%C 8.58%H 5.66%N
Found:
63.52%C 8.62%H 5.30%N

EXAMPLE 8

7β-(N, N-Diethylcarbamoyloxy)-8, 13-epoxy-1α, 6β, 9α-trihydroxylabd-14-en-11-one-1, 9-dimethylformamide acetal

To a stirred solution of 1.0 g of 8, 13-epoxy-1α, 6β, 7β, 9α-tetrahydrolabd-14-en-11-one-1, 9-dimethylformamide acetal in 100 ml of dry tetrahydrofuran under nitrogen at 0° was added 2.83 ml of 1M lithium bis(trimethylsilyl) amide in tetrahydrofuran. The solution was stirred 20 min at 0°. To the solution was added 0.62 ml (0.66 g) of N, N-diethylcarbamoyl chloride. The solution was heated to ambient temperature, then to reflux and reflux was continued for 24 hr. The solution was allowed to cool to ambient temperature, poured into ice/water, diluted with ether, washed three times with water, once with saturated sodium chloride solution, and dried over anhydrous sodium sulfate. Filtration followed by evaporation of solvent provided an oil. The oil was dissolved in a minimum volume of 30% n-butyl acetate/hexane and flash chromatographed on silica gel. The column was eluted with 30% n-butyl acetate/hexane, 40% n-butyl acetate/hexane and 50% n-butyl acetate/hexane. The appropriate fraction was concentrated and the residue was crystallized from cyclohexane to provide 24 mg (18.9%) of product.
ANALYSIS:
Calculated for $C_{28}H_{46}N_2O_7$:
64.34%C 8.87%H 5.36%N
Found:
64.50%C 8.97%H 5.25%N

EXAMPLE 9

8, 13-Epoxy-7β-(N-hydroxylaminocarbonyloxy)-1α, 6β, 9α-trihydroxylabd-14-en-11-one-1, 9-dimethylformamide acetal

8, 13-Epoxy-1α, 6β, 7β, 9α-tetrahydrolabd-14-en-11-one-1, 9-dimethylformamide acetal (500 mg) was dissolved in 10 ml of dichloromethane together with 202.5 mg of 1,1'-carbonyldiimidazole under nitrogen.

The mixture was stirred at ambient temperature overnight. Hydroxylamine hydrochloride (1.6 g) was dissolved in 25 ml of methanol together with a single crystal of phenophthalein. Sufficient 25% sodium methoxide in methanol was added to turn the color of the solution pink. The sodium chloride was allowed to settle. An aliquot of 6 ml of the hydroxylamine solution in methanol was added to the original solution and the mixture was stirred for 1 hr. The mixture was diluted with chloroform and extracted twice with water, once with 0.01 N hydrochloric acid and one with dilute sodium bicarbonate. The organic phase was dried over anhydrous sodium sulfate, filtered, and evaporated. The residue was flash chromatographed on silica gel in hexane:ethyl acetate (1:1), followed by hexane:ethyl acetate (1:2). The appropriate fractions were combined and evaporated and the residue was crystallized from hexane:ether to yield 200 mg (35.2%) of product, mp 119-139°.

ANALYSIS:

Calculated for $C_{24}H_{38}N_2O_8$:

59.72%C 7.95%H 5.80%N

Found:

59.46%C 8.09%H 5.41%N

EXAMPLE 10

8, 13-Epoxy-7β-(N-methyl-N-hydroxylaminocarbonyloxy)-1α, 6β, 9α-trihydroxylabd-14-en-11-one-1, 9-dimethylformamide acetal

8, 13-Epoxy-1α, 6β, 7β, 9α-tetrahydroxylabd-14-en-11-one-1, 9-dimethylformamide acetal (500 mg) was dissolved in 10 ml of dichloromethane together with 202.5 mg of 1,1'-carbonyldiimidazole under nitrogen. The mixture was stirred at ambient temperature overnight. N-Methylhydroxylamine hydrochloride (3.6 g) was dissolved in 25 ml of methanol together with a single crystal of phenophthalein. Sufficient 25% sodium methoxide in methanol was added to change the color of the solution pink. The sodium chloride was allowed to settle out. An aliquot of 6 ml of the N-methylhydroxylamine in methanol solution was added to the original solution and the mixture was stirred for 1 hr. The mixture was diluted with chloroform and the solution was washed twice with water, once with 0.01 N hydrochloric acid and once with dilute sodium bicarbonate solution. The organic phase was dried over anhydrous sodium sulfate, filtered and evaporated. The residue was flash chromatographed on silica gel in hexane:ethyl acetate (2:1) (1 ℓ) and hexane/ethyl acetate (1:1). The appropriate fractions were combined and evaporated. The residue was crystallized from hexane:ether to yield 200 mg (34.2%) of product, mp 105-119°.

ANALYSIS:

Calculated for $C_{25}H_{40}N_2O_5$:

60.46%C 8.13%H 5.64%N

Found:

60.75%C 8.54%H 5.29%N

EXAMPLE 11

8, 13-Epoxy-7β-(N-ethylaminocarbonyloxy)-1α, 6β, 9α-trihydroxylabd-14-en-11-one-1, 9-dimethylformamide acetal

To a solution of 1.0 g of 8, 13-epoxy-1α, 6β, 7β, 9α-tetrahydrolabd-14-en-11-one-1, 9-dimethylformamide acetal in 100 ml of dry tetrahydrofuran was added 0.237 ml of 1M lithium bis(trimethylsilyl) amide in tetrahydrofuran. The solution was stirred for 0.5 hr at ambient temperature. To the solution was added 0.336 g of ethyl isocyanate. The solution was stirred at reflux under nitrogen for 18 hr and allowed to cool to ambient temperature. The solution was diluted with ethyl acetate, poured into ice/water, extracted twice with ethyl acetate, washed with water, saturated sodium chloride solution and dried over anhydrous sodium sulfate. Filtration followed by evaporation of solvent provided an oil. The oil was dissolved in a minimum volume of 40% ethyl acetate;hexanes and flash chromatographed on silica gel using the same solvent system. Evaporation of solvent from the appropriate fractions provided 747 mg (63.5%) of product.

ANALYSIS:

Calculated for $C_{26}H_{42}N_2O_7$:
63.13%C 8.56%H 5.67%N
Found:
62.90%C 8.88%H 5.47%N

EXAMPLE 12

8, 13-Epoxy-7β-(N-methoxylaminocarbonyloxy)-1α, 6β, 9α-trihydroxylabd-14-en-11-one-1, 9-dimethylformamide acetal

8, 13-Epoxy-1α, 6β, 7β, 9α-tetrahydroxylabd-14-en-11-one-1, 9-dimethylformamide acetal (500 mg) was dissolved in 10 ml of dichloromethane together with 202.5 mg of 1,1'-carbonyldiimidazole, and the mixture was stirred under nitrogen at ambient temperature overnight. Methoxylamine hydrochloride (3.6 g) was dissolved in 25 ml of methanol together with a crystal of phenophthalein and sufficient 25% sodium methoxide in methanol was added to change the color of the solution to just pink. A 6.0 ml-aliqnot of the methoxylamine solution was added to the orginal mixture and the resultant mixture was stirred under nitrogen at ambient temperature overnight. The reaction mixture was evaporated and the residue was flash chromatographed on silica gel in hexane:ethyl acetate (2:1). Evaporation of the appropriate fractions gave 144.3 mg (24.7%) of product, as an amorphous solid, mp 85-92°.
ANALYSIS:
Calculated for $C_{25}H_{40}N_2O_8$:
60.46%C 8.13%H 5.64%N
Found:
60.24%C 8.42%H 5.41%N

EXAMPLE 13

8, 13-Epoxy-7β-(1-pyrrolidinocarbonyloxy)-1α, 6β, 9α-trihydroxylabd-14-en-11-one-1, 9-dimethylformamide acetal

A solution of 500 mg of 8, 13-epoxy-1α, 6β, 7β, 9α-tetrahydroxylabd-14-en-11-one-1, 9-dimethylformamide acetal in 50 ml of tetrahydrofuran containing 202.5 mg of 1,1'-carbonyldiimidazole was stirred at ambient temperature under nitrogen overnight. Pyrrolidine 417μ1 (355 mg) was added and the mixture was stirred under nitrogen at ambient temperature for 3 hr and allowed to stand at ambient temperature for two and one-half days. The mixture was quenched with water and evaporated. The residue was dissolved in ether. The mixture was washed with water, dilute potassium carbonate solution, dried over anhydrous sodium sulfate and filtered. The filtrate was evaporated and the residue was flash chromatographed on silica gel in hexane:ethyl acetate (1:1). The appropriate fractions were combined and the residue was crystallized from hexane-ether to provide 30 mg (4.81%) of product, mp 218-220°.

EXAMPLE 14

7β-(Aminocarbonyloxy)-8, 13-epoxy-1α, 6β, 9α-trihydroxylabd-14-en-11-one

A solution of 128.4 mg of 7β-(aminocarbonyloxy)-8, 13-epoxy-1α, 6β, 9α-trihydroxy-14-en-11-one-1, 9-dimethylformamide acetal in 2 ml of 80% acetic acid and 2 ml of methanol was stirred at ambient temperature for 36 hrs. The mixture was evaporated and the residue was flash chromatographed on silica gel in hexane:ethyl acetate (1:1). The appropriate fractions were combined and evaporated. The residue was crystallized from hexane:ether to afford 50 mg (44.2%) of product, mp 124-145°.
ANALYSIS:
Calculated for $C_{21}H_{33}NO_7$:
61.28%C 8.10%H 3.40%N
Found:
61.09%C 7,99%H 3.35%N

EXAMPLE 15

8, 13-Epoxy-7$\beta$-(N-methylaminocarbonyloxy)-1$\alpha$, 6$\beta$, 9$\alpha$-trihydroxylabd-14-en-11-one

A solution of 291.6 mg of 8, 13-epoxy-7$\beta$-(N-methylaminocarbonyloxy)-1$\alpha$, 6$\beta$, 9$\alpha$-trihydroxylabd-14-en-11-one1, 9-dimethylformamide acetal in 6 ml of methanol:80% acetic acid (1:1) was stirred overnight under nitrogen at ambient temperature. The mixture was evaporated. The residue was suspended in ethyl acetate washed with aqueous sodium bicarbonate solution and evaporated. The residue was flash chromatographed on silica gel in hexane:ethyl acetate (1:1). The appropriate fractions were combined and evaporated. The residue was crystallized from hexane:ether to afford 67.4 mg (54.4%) of product, mp 133-154°.

ANALYSIS:

Calculated for $C_{22}H_{35}NO_7$:

62.09%C 8.31%H 3.29%N

Found:

61.35%C 8.08%H 3.17%N

EXAMPLE 16

8, 13-Epoxy-7$\beta$-(4-morpholinocarbonyloxy)-1$\alpha$, 6$\beta$, 9$\alpha$-trihydroxylabd-14-en-11-one

A solution of 265.0 mg of 8, 13-epoxy-7$\beta$-(4-morpholinocarbonyloxy)-1$\alpha$, 6$\beta$, 9$\alpha$-trihydroxylabd-14-en-11-one-1, 9-dimethylformamide acetal in a mixture of 2 ml of 80% acetic acid and 2 ml of methanol was stirred at ambient temperature for 36 hr. The mixture was evaporated and the residue was flash chromatographed on silica gel in hexane:ethyl acetate (1:1). The approriate fractions were combined and evaporated. The residue was crystallized from hexane:ether to provide 158.2 mg (65.8%) of product, mp 180-188°.

ANALYSIS:

Calculated for $C_{25}H_{39}NO_8$:

62.34%C 8.18%H 2.91%N

Found:

62.33%C 8.11%H 2.80%N

EXAMPLE 17

8, 13-Epoxy-7$\beta$-(4-hydroxy-1-piperidinocarbonyloxy)-1$\alpha$, 6$\beta$, 9$\alpha$-trihydroxylabd-14-en-11-one

A solution of 306.1 mg of 8, 13-epoxy-7$\beta$-(4-hydroxy-1-piperidinocarbonyloxy)-1$\alpha$, 6$\beta$, 9$\alpha$-trihydroxylabd-14-en-11-one-1, 9-dimethylformamide acetal in 2 ml of 80% acetic acid and 2 ml of methanol was stirred for 36 hr at ambient temperature. The solvent was evaporated and the residue was flash chromatographed on silica gel in hexane:ethyl acetate (1:2). The appropriate fractions were combined and evaporated. The residue was crystallized from hexane:ether to afford 185.1 mg (67.2%) of product, mp 145-156°.

ANALYSIS:

Calculated for $C_{26}H_{41}NO_8$:

63.00%C 8.35%H 2.82%N

Found:

62.71%C 8.40%H 2.79%N

EXAMPLE 18

8, 13-Epoxy-7$\beta$-(1-pyrrolidinocarbonyloxy)-1$\alpha$, 6$\beta$, 9$\alpha$-trihydroxylabd-14-en-11-one

A solution of 300 mg 8, 13-epoxy-7$\beta$-(1-pyrrolidinocarbonyloxy)-1$\alpha$, 6$\beta$, 9$\alpha$-trihydroxylabd-14-en-11-one-1, 9-dimethylformamide acetal in 5 ml of 80% aqueous acetic acid and 5 ml of methanol was stirred at ambient temperature for 18 hr under nitrogen. The solvent was evaporated under vacuum. The residue was

suspended in ethyl acetate and washed with saturated aqueous sodium bicarbonate solution. The ethyl acetate layer was evaporated and the residue was flash chromatographed on silica gel in hexane:ethyl acetate (2:1). The appropriate fractions were combined and evaporated. The residue was crystallized from hexane:ethyl acetate to afford 88.1 mg (31.6%) of product, mp 230-233°.

ANALYSIS:

Calculated for $C_{25}H_{39}NO_7$:

64.48%C 8.46%H 3.01%N

Found:

64.44%C 8.48%H 3.15%N

EXAMPLE 19

8, 13-Epoxy-7β-(2-hydroxyethylaminocarbonyloxy)-1α, 6β, 9α-trihydroxylabd-14-en-11-one

8, 13-Epoxy-7β-(2-hydroxyethylaminocarbonyloxy)-1α, 6β, 9α-trihydroxylabd-14-en-11-one-1, 9-dimethylformamide acetal (200 mg) was dissolved in a mixture of 2 ml of methanol and 2 ml of 80% acetic acid and stirred at ambient temperature under nitrogen for 3 days. The mixture was evaporated and the residue was flash chromatographed on silica gel in hexane:acetone (2:1). The appropriate fractions were combined, evaporated and crystallized from hexane:ethyl acetate to afford 92.4 mg (51.8%) of product, mp 116-120°.

ANALYSIS:

Calculated for $C_{23}H_{37}NO_5$:

60.63%C 8.20%H 3.07%N

Found:

60.47%C 8.44%H 3.14%N

EXAMPLE 20

7β-(N-2, 3-Dihydroxypropylaminocarbonyloxy)-8, 13-epoxy-1α, 6β, 9α-trihydroxylabd-14-en-11-one

7β-(N-2, 3-Dihydroxypropylaminocarbonyloxy)-8, 13-epoxy-1α, 6β, 9α-trihydroxylabd-14-en-11-one-1, 9-dimethylformamide acetal (200 mg) was dissolved in a mixture of 2 ml of methanol and 2 ml of 80% acetic acid. The mixture was stirred under nitrogen at ambient temperature for 4 days. The mixture was evaporated and the residue flash chromatographed on silica gel in hexane:acetone (1:1). The appropriate fractions were combined and evaporated. The residue was crystallized from hexane:n-butyl acetate to afford 74.8 mg (41.6%) of product, mp 90-105°.

ANALYSIS:

Calculated for $C_{24}H_{39}NO_9$:

59.35%C 8.11%H 2.88%N

Found:

58.89%C 8.29%H 2.71%N

EXAMPLE 21

8, 13-Epoxy-7β-(N-hydroxylaminocarbonyloxy)-1α, 6β, 9α-trihydroxy-labd-14-en-11-one

8, 13-Epoxy-1α, 6β, 7β, 9α-trihydroxylabd-14-en-11-one-1, 9-dimethylformamide acetal (1 g) was dissolved in 20 ml of dry dichloromethane together with 405 mg of 1,1′-carbonyldiimidazole, and the mixture was stirred under nitrogen overnight. Hydroxylamine (303.6 mg) generated from the hydrochloride in methanol by adding methanolic sodium methoxide was added to the original solution, and the resultant mixture was stirred overnight. The mixture was evaporated and the residue was flash chromatographed on silica gel in hexane:ethyl acetate (1:2). The fractions were combined and the residue was dissolved in 10 ml of methanol and 1 ml of 2 N hydrochloric acid, and the mixture was stirred at ambient temperature under nitrogen overnight. The mixture was evaporated. The residue was partitioned between saturated aqueous sodium bicarbonate and ethyl acetate. The ethyl acetate phase was dried over anhydrous sodium sulfate,

filtered and evaporated. The residue was flash chromatographed on silica gel in hexane:ethyl acetate (1:1). The appropriate fractions were combined and evaporated. The residue was crystallized from hexane:ether to afford 1.52.0 mg (15.1%) of product, mp 209-211°.

ANALYSIS:

Calculated for $C_{21}H_{33}NO_8$:

58.99%C 7.80%H 3.27%N

Found:

58.85%C 8.08%H 3.24%N

EXAMPLE 22

8, 13-Epoxy-7β-(N-methylhydroxylaminocarbonyloxy)-1α, 6β, 9α-trihydroxylabd-14-en-11-one

8, 13-Epoxy-7β-(N-methylhydroxylaminocarbonyloxy)-1α, 6β, 9α-trihydroxylabd-14-en-11-one-1, 9-dimethylformamide acetal (200 mg) was dissolved in 2 ml of 80% acetic acid and 2 ml of methanol. The mixture was stirred at ambient temperature under nitrogen for 36 hr and evaporated. The residue was dissolved in chloroform and extracted with saturated aqueous sodium bicarbonate solution. The organic phase was loaded onto a flash chromatography column of silica gel packed in hexane:ethyl acetate (2:1) and eluted with the same solvent mixture. The appropriate fractions were combined and evaporated. The residue was crystallized from hexane:ether to afford 90 mg of (50.6%) of product, mp 110-145°.

ANALYSIS:

Calculated for $C_{22}H_{35}NO_8$:

59.84%C 7.99%H 3.17%N

Found:

59.77%C 7.94%H 2.94%N

EXAMPLE 23

8, 13-Epoxy-7β-(N-ethylaminocarbonyloxy)-1α, 6β, 9α-trihydroxylabd-14-en-11-one

A solution of 0.742 mg of 8, 13-epoxy-7β-(N-ethylaminocarbonyloxy)-1α, 6β, 9α-trihydroxylabd-14-en-11-one-1, 9-dimethylformamide acetal in 10 ml of methanol and 10 ml of 80% acetic acid was stirred at room temperature for 48 hr. The solution was diluted with ethyl acetate, washed three times with water and once with saturated sodium chloride solution. The solution was dried over anhydrous sodium sulfate, filtered and concentrated. The residue was dissolved in 1:1 n-butyl acetate/hexanes and flash chromatographed on silica gel. The initial fractions were combined and concentrated. The residue crystallized to provide 109 mg of product, mp 165-167°. Subsequent fractions were combined and concentrated to provide an additional 163 mg of product. The total yield of product was 272 mg (41.3%).

ANALYSIS:

Calculated for $C_{23}H_{37}NO_7$:

62.85%C 8.45%H 3.15%N

Found:

62.79%C 8.47%H 3.07%N

EXAMPLE 24

8, 13-Epoxy-7-β (N-methoxylaminocarbonyloxy)-1α, 6β, 9α-trihydroxylabd-14-en-11-one

8, 13-Epoxy-7' b-(N-methoxylaminocarbonyloxy)-1α, 6β, 9α-trihydroxylabd-14-en-11-one-1, 9-dimethylformamide acetal (121.5 mg) was dissolved in 2 ml of 80% acetic acid together with 2 ml of methanol. The mixture was stirred at ambient temperature under nitrogen for 36 hr. The solvent was evaporated and the residue was flash chromatographed on silica gel eluting with hexane:ethyl acetate (65:35). The appropriate fractions were combined and evaporated. Crystallization of the residue from hexane:ether provided 40 mg (37%) of product, mp 100-120°.

ANALYSIS:

Calculated for $C_{22}H_{35}NO_8$:
59.84%C 7.99%H 3.17%N
Found:
60.03%C 8.20%H 2.94%N

EXAMPLE 25

8, 13-Epoxy-7β-[N, N-bis-2-hydroxyethylaminocarbonyloxy)-1α, 6β, 9α-trihydroxylabd-14-en-11-one-1, 9-dimethylformamide acetal

To a stirred solution of 2.0 g of 8, 13-epoxy-1α, 6β, 7β, 9α-tetrahydroxylabd-14-en-11-one-1, 9-dimethylformamide acetal in 30 ml of dry ethyl acetate under nitrogen was added 0.814 g of 1,1'-carbonyldiimidazole. The solution was stirred at room temperature overnight. To the solution was added 2 ml of N, N-diethanolamine. The solution was stirred 24 hr at ambient temperature and flash chromatographed on silica gel. The column was eluted with 1/1 ethyl acetate/hexanes, 2/1 ethyl acetate/hexanes and ethyl acetate. The appropriate fractions were combined and concentrated to provide 0.34 g (17%) of product, as a foam, mp 97-119°.
ANALYSIS:
Calculated for $C_{28}H_{46}N_2O_9$:
60.63%C 8.36%H 5.05%N
Found:
60.90%c 8.28%H 4.97%N

EXAMPLE 26

8, 13-Epoxy-6β-(N-methylaminocarbonyloxy)-1α, 7β, 9α-trihydroxylabd-14-en-11-one

To a solution of 0.3 g of 8, 13-epoxy-7β-(N-methylaminocarbonyloxy)-1α, 6β, 9α-trihydroxylabd-14-en-11-one in 10 ml of t-butanol and 1 ml of dry tetrahydrofuran was added 1.1 g of potassium t-butoxide. The solution was stirred for 1 hr at room temperature, poured into ice/water, washed twice with water, once with saturated sodium chloride solution and dried over anhydrous sodium sulfate. Filtration followed by evaporation of solvent provided an oil. The oil was dissolved in a minimum volume of 1:1 ethyl acetate:hexanes and chromatographed on silica gel eluting with 1:1 ethyl acetate:hexanes, followed by 3:2 ethyl acetate:hexanes. The appropriate fractions were combined and concentrated to an oil, which crystallized on standing to provide, after drying at 111° (1 mm), 180 mg (60%) of product, mp 242-243°.
ANALYSIS:
Calculated for $C_{22}H_{35}NO_7$:
62.09%C 8.29%H 3.29%N
Found:
62.29%C 8.24%h 2.87%N

EXAMPLE 27

8, 13-Epoxy-1α-(1-pyrrolidinocarbonyloxy)-6β, 7β, 9α-trihydroxylabd-14-en-11-one

A solution of 100 mg of 8, 13-epoxy-1α, 6β, 7β, 9α-tetrahydroxylabd-14-en-11-one was dissolved in 10 ml of dry tetrahydrofuran containing 44 mg of 1,1'carbonyldiimidazole was stirred at ambient temperature under nitrogen for 1.5 hr. Pyrrolidine (83 1, 71 mg) was added and the mixture was stirred overnight. The mixture was evaporated and the residue was suspended in ether, washed with dilute aqueous hydrochloric acid, water and dilute aqueous potassium carbonate. The ether phase was dried over anhydrous sodium sulfate, filtered and evaporated. The residue was flash chromatographed on silica gel in hexane:ethyl acetate (2:1). The appropriate fractions were combined and evaporated. The residue was recrystallized from cyclohexane:ethyl acetate to afford 40.0 mg (31.6%) of product, mp 207-210°.
ANALYSIS:

Calculated for $C_{25}H_{39}NO_7$:

64.48%C 8.46%H 3.01%N

Found:

64.62%C 8.68%H 3.12%N

REACTION SCHEME A

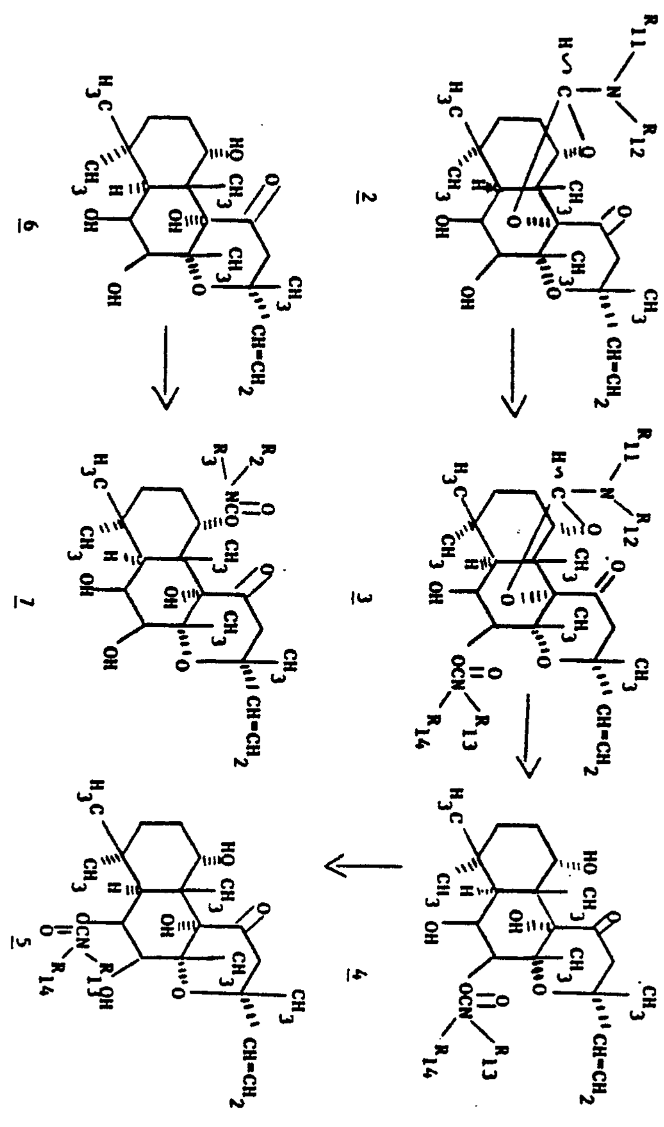

wherein $R_2$, $R_3$, $R_{11}$, $R_{12}$, $R_{13}$ and $R_{14}$ are as hereinbeforedescribed.

REACTION SCHEME B

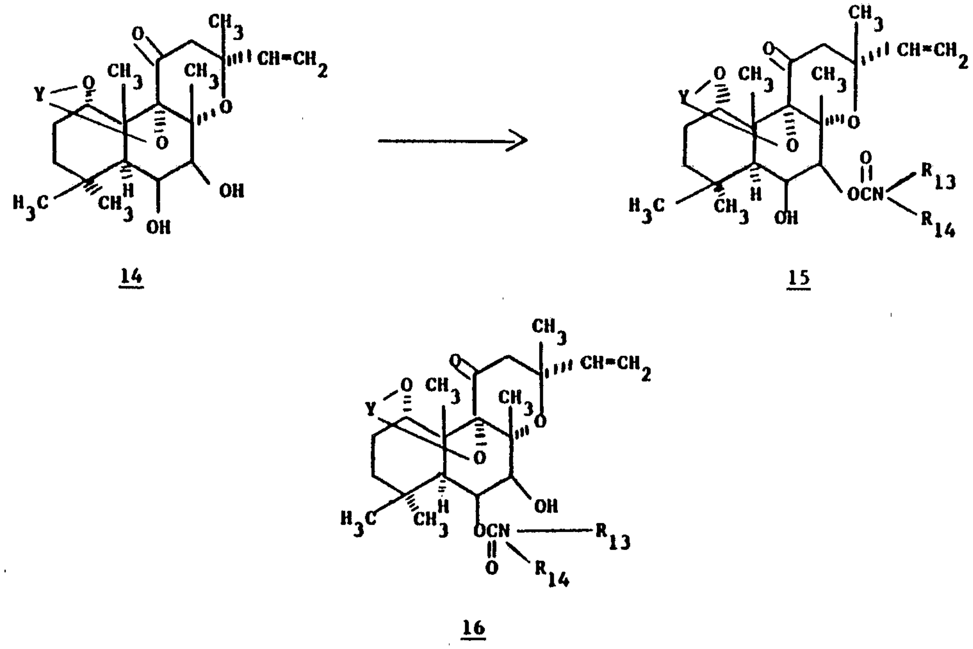

14

15

16

wherein $R_{13}$, $R_{14}$, and Y are as hereinbeforedescribed.

**Claims**

1. A compound of the formula 1

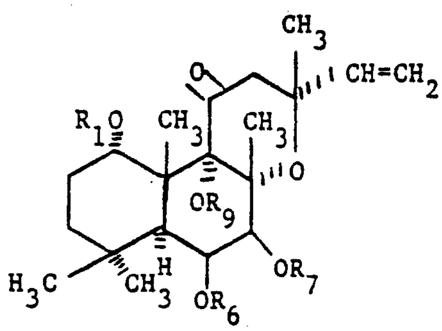

1

wherein:

(a) $R_1$ is hydrogen or a group of the formula $R_2R_3N\overset{\overset{O}{\|}}{C}$ wherein $R_2$ and $R_3$ are independently hydrogen or loweralkyl of 1 to 6 carbon atoms; or $R_2$ and $R_3$ taken together with the nitrogen atom to which they are attached form a group of the formula

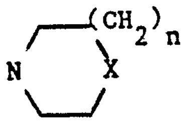

wherein X is O, S or a group of the $CHR_4$ wherein $R_4$ is hydrogen, loweralkyl, a group of the formula $OR_5$ wherein $R_5$ is hydrogen, loweralkyl or a group of the formula $\overset{\overset{O}{\|}}{C}R_{10}$ wherein $R_{10}$ is loweralkyl and n is 0 or 1;

(b) $R_9$ is hydrogen;

(c) $R_1$ and $R_9$ taken together form a group of the formula CO, a group of the formula SO or a group of the formular $CHNR_{11}R_{12}$ wherein $R_{11}$ and $R_{12}$ are each independently loweralkyl of 1 to 6 carbon atoms; or $R_{11}$ and $R_{12}$ taken together with the nitrogen atom to which they are attached form a group of the formula

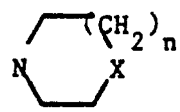

wherein X and n are as above;

(d) $R_6$ and $R_7$ independently are hydrogen or a group of the formula $R_{13}R_{14}N\overset{\overset{O}{\|}}{C}$ wherein $R_{13}$ is hydrogen, loweralkyl of 1 to 6 carbon atoms, a group of the formula $R_{15}O(CH_2)_p$wherein $R_{15}$ is hydrogen or loweralkyl of 1 to 6 carbon atoms and p is 0, 2 or 3 or a group of the formular $HOCH_2CH(OH)CH_2$; $R_{14}$ is hydrogen, loweralkyl of 1 to 6 carbon atoms, a group of the formula $R_{16}O(CH_2)_q$ wherein $R_{16}$ is hydrogen and $_q$ is 2 or 3; or $R_{13}$ and $R_{14}$ taken together with the nitrogen atom to which they are attached form a group of the formula

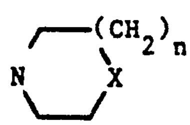

wherein X and n are as above; or the optical and geometric isomers thereof; with the provisos:

(e) that $R_1$, $R_6$ and $R_7$ are not simultaneously hydrogen,

(f) that when $R_1$ and $R_6$ are hydrogen, $R_{13}$ or $R_{14}$ are not simultaneously loweralkyl of 1 to 6 carbon atoms, and

(g) that then wen $R_1$ and $R_9$ taken together form a group of the formula CO, SO, or $CHNR_{11}R_{12}$, $R_6$ and $R_7$ are not simultaneously hydrogen.

2. A compound according to claim 2 wherein $R_1$ is hydrogen and $R_7$ is a group of the formula $R_{13}R_{14}N\overset{O}{\overset{\|}{C}}$ wherein $R_{13}$ and $R_{14}$ are as above.

3. The compound according to claim 2 which is 7β-(aminocarbonyloxy)-8, 13-epoxy-lα, 6β, 9α-trihydroxylabd-14-en-11-one.

4. The compound according to claim 2 which is 8, 13-epoxy-7β-(N-methylaminocarbonyloxy)-1α, 6β, 9α-trihydroxylabd-14-en-11-one.

5. The compound according to claim 2 which is 8, 13-epoxy-7β-(2-hydroxyethylaminocarbonyloxy) -1α, 6β, 9α-trihydroxylabd-14-en-11-one.

6. A pharmaceutical composition which comprises as the active ingredient a compound as defined in claim 1 in association with a pharmaceutically acceptable carrier and/or auxiliary.

7. Use of a compound as defined in claim 1 for the preparation of a medicament having intraocular pressure reducing acitivity.

8. A process for the preparation of a compound of the formula 1

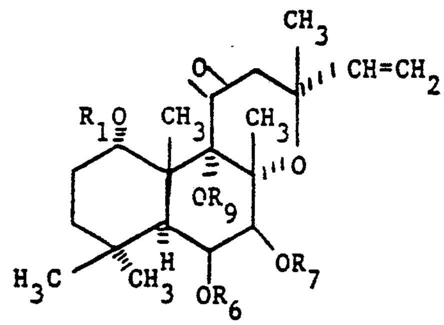

1

wherein:

(a) $R_1$ is hydrogen or a group of the formula $R_2R_3N\overset{O}{\overset{\|}{C}}$ wherein $R_2$ and $R_3$ are independently hydrogen or loweralkyl of 1 to 6 carbon atoms; or $R_2$ and $R_3$ taken together with the nitrogen atom to which they are attached form a group of the formula

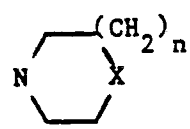

wherein X is O, S or a group of the $CHR_4$ wherein $R_4$ is hydrogen, loweralkyl, a group of the formula $OR_5$ wherein $R_5$ is hydrogen, loweralkyl or a group of the formula $\overset{O}{\overset{\|}{C}}R_{10}$ wherein $R_{10}$ is loweralkyl and n is 0 or 1;

(b) $R_9$ is hydrogen;

(c) $R_1$ and $R_9$ taken together form a group of the formula CO, a group of the formula SO or a group of the formula $CHNR_{11}R_{12}$ wherein $R_{11}$ and $R_{12}$ are each independently loweralkyl of 1 to 6 carbon atoms; and $R_{11}$ or $R_{12}$ taken together with the nitrogen atom to which they are attached form a group of the formula

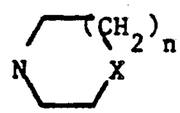

wherein X and n are as above;

(d) $R_6$ and $R_7$ independently are hydrogen or a group of the formula $R_{13}R_{14}N\overset{O}{\overset{\|}{C}}$ wherein $R_{13}$ is hydrogen, loweralkyl of 1 to 6 carbon atoms, a group of the formula $R_{15}O(CH_2)_p$wherein $R_{15}$ is hydrogen or loweralkyl of 1 to 6 carbon atoms and p is 0, 2 or 3 or a group of the

formula $HOCH_2CH(OH)CH_2$; $R_{14}$ is hydrogen, loweralkyl of 1 to 6 carbon atoms, a group of the formula $R_{16}O-(CH_2)_q$ wherein $R_{16}$ is hydrogen and q is 2 or 3; and $R_{13}$ or $R_{14}$ taken together with the nitrogen atom to which they are attached form a group of the formula

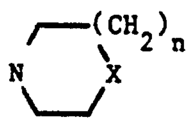

wherein X and n are as above; or the optical and geometric isomers thereof; with the provisos:

(e) that $R_1$, $R_6$ and $R_7$ are not simultaneously hydrogen,

(f) that when $R_1$ and $R_6$ are hydrogen, $R_{13}$ or $R_{14}$ are not simultaneously loweralkyl of 1 to 6 carbon atoms, and

(g) that then wen $R_1$ and $R_9$ taken together form a group of the formular CO, SO, or $CHNR_{11}R_{12}$, $R_6$ and $R_7$ are not simultaneously hydrogen which comprises

a) reacting a compound of the formula 2

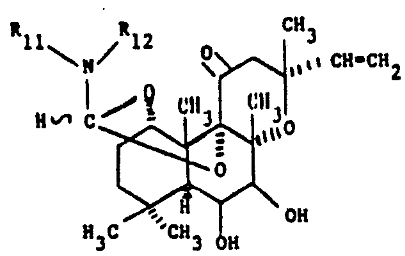

2

wherein $R_{11}$ and $R_{12}$ are as defined above, with 1,1′-carbonyldiimidazole to afford a compound of formula 9

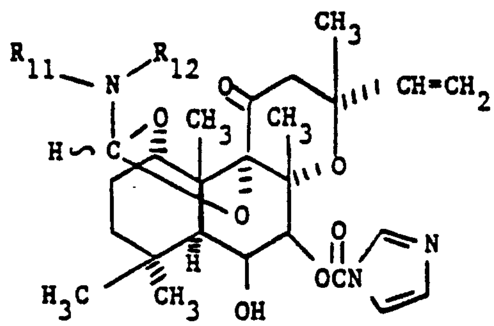

9

treating the same after or without isolation with an amine of the formula $HNR_{13}R_{14}$, wherein $R_{13}$ and $R_{14}$ are as defined above, to afford a compound of the formula 3

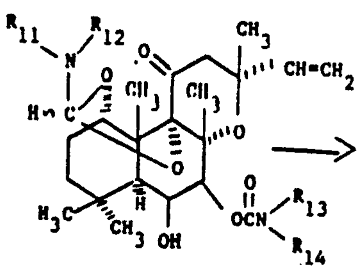

3

and hydrolyzing a compound of the formula 3 with an aqueous alkanol, an aqueous alkanoic acid in alkanol or a mineral acid in aqueous alkanol to afford a compound of the formula 4

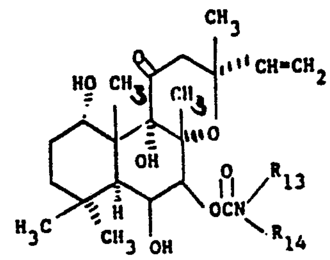

4

or

b) treating a compound of the formula 2 with a compound of the formula 11

$[(R_{17})_3Si]_2NM$ 11

where $R_{17}$ is loweralkyl and M is an alkali metal, to afford an alkali metal alkoxide of the compound of formula 2, and

$b_1$) treating the compound obtained with either an isocyanate of formula $R_{13}NCO$ or a carbamoyl halide of formula 13

$R_{13}R_{14}N\overset{O}{\overset{\|}{C}}$ Hal 13

where $R_{13}$ and $R_{14}$ are as defined above, to afford a compound of the formula 3, and

$b_2$) hydrolyzing a compound of the formula 3 as described above, to afford a compound of the formula 4,

c) optionally rearranging a compound of the formula 4 by means of an alkali metal alkoxide to afford a compound of the formula 5

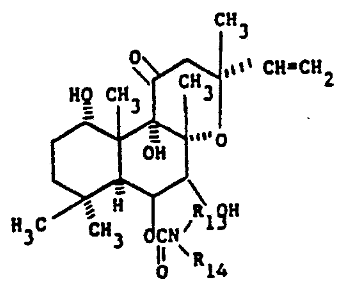

5

wherein $R_{13}$ and $R_{14}$ are as defined above,

d) optionally reacting a compound of the formula 6

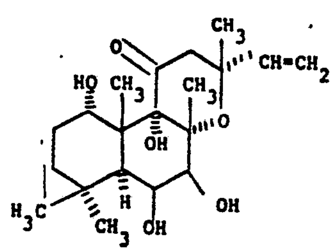

6

with 1,1'-carbonyldiimidazole, followed by the reaction with an amine of the formula $HNR_{13}R_{14}$, where $R_{13}$ and $R_{14}$ are as defined above, according to step a) above to afford a compound of the formula 7

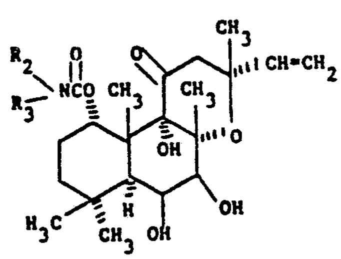

7

wherein $R_2$ and $R_3$ are as defined,
e) optionally reacting a compound of the formula 14

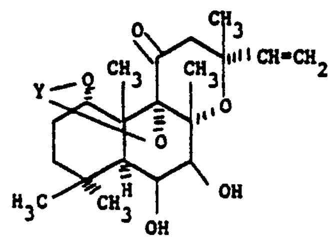

14

wherein Y is SO or CO, with 1,1'-carbonyldiimidazole, followed by the treatment with an amine of the formula $HNR_{13}R_{14}$, wherein $R_{13}$ and $R_{14}$ are as defined above, to afford a compound of the formula 2

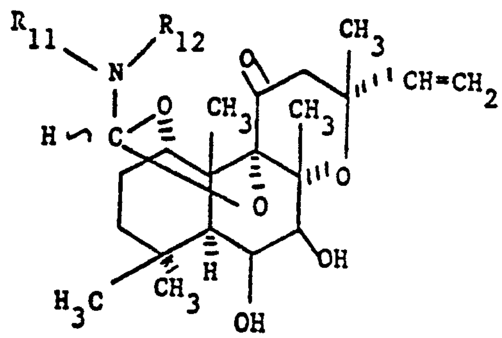

2

or
$e_1$) reacting an alkoxid of a compound of the formula 14 with isocyanate or carbamoyl halide according to step $b_1$) above to afford a compound of the formula 15,

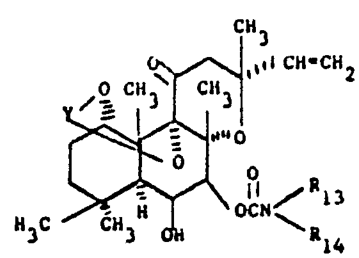

15

and
f) optionally rearranging a compound of the formula 15 according to step c) to afford a compound of the formula 16

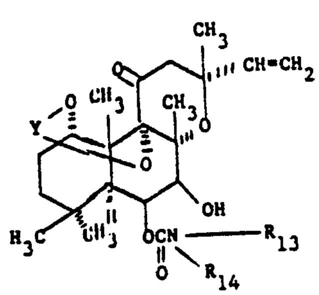

16

wherein $R_{13}$, $R_{14}$ and Y are as defined above.
9. A compound of the formula

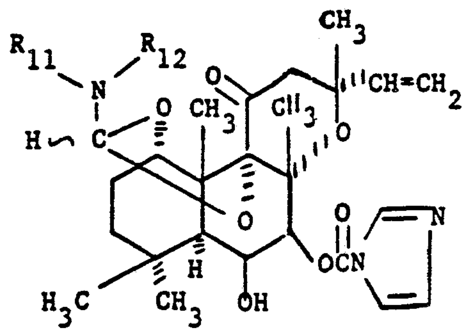

wherein $R_{11}$ and $R_{12}$ are each independently loweralkyl of 1 to 6 carbon atoms; and $R_{11}$ and $R_{12}$ taken together with the nitrogen atom to which they are attached form a group of the formula

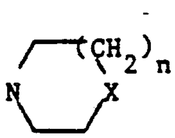

wherein X is O, S or a group of the formula $CHR_4$ wherein $R_4$ is hydrogen, loweralkyl of 1 to 6 carbon atoms, or a group of the formula $OR_5$ wherein $R_5$ is hydrogen, loweralkyl of 1 to 6 carbon atoms or a group of the formula $\overset{O}{\overset{\|}{C}}R_{10}$ wherein $R_{10}$ is loweralkyl of 1 to 6 carbon atoms and n is O or 1.

<u>Claims for the following contracting States: ES, GR</u>

1. A process for the preparation of a compound of the formula <u>1</u>

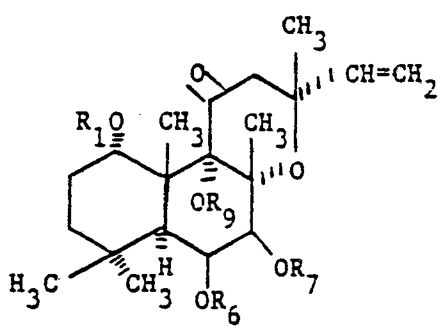

1

wherein:

(a) $R_1$ is hydrogen or a group of the formula $R_2R_3N\overset{O}{\overset{\|}{C}}$ wherein $R_2$ and $R_3$ are independently hydrogen or loweralkyl of 1 to 6 carbon atoms; or $R_2$ and $R_3$ taken together with the nitrogen atom to which they are attached form a group of the formula

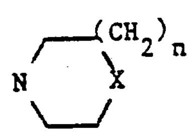

wherein X is O, S or a group of the $CHR_4$ wherein $R_4$ is hydrogen, loweralkyl, a group of the formula $OR_5$ wherein $R_5$ is hydrogen, loweralkyl or a group of the formula $\overset{O}{\overset{\|}{C}}R_{10}$ wherein $R_{10}$ is loweralkyl and n is 0 or 1;

(b) $R_9$ is hydrogen;

(c) $R_1$ and $R_9$ taken together form a group of the formula CO, a group of the formula SO or a group of the formula $CHNR_{11}R_{12}$ wherein $R_{11}$ and $R_{12}$ are each independently loweralkyl of 1 to 6 carbon atoms; and $R_{11}$ or $R_{12}$ taken together with the nitrogen atom to which they are attached form a group of the formula

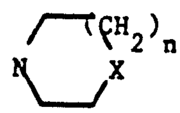

wherein X and n are as above;

(d) $R_6$ and $R_7$ independently are hydrogen or a group of the formula $R_{13}R_{14}N\overset{O}{\overset{\|}{C}}$ wherein $R_{13}$ is hydrogen, loweralkyl of 1 to 6 carbon atoms, a group of the formula $R_{15}O(CH_2)_p$wherein $R_{15}$ is hydrogen or loweralkyl of 1 to 6 carbon atoms and p is 0, 2 or 3 or a group of the formula $HOCH_2CH(OH)CH_2$; $R_{14}$ is hydrogen, loweralkyl of 1 to 6 carbon atoms, a group of the formula $R_{16}O$-$(CH_2)_q$ wherein $R_{16}$ is hydrogen and q is 2 or 3; or $R_{13}$ and $R_{14}$ taken together with the nitrogen atom to which they are attached form a group of the formula

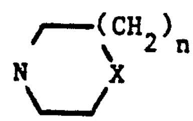

wherein X and n are as above; or the optical and geometric isomers thereof; with the provisos:

(e) that $R_1$, $R_6$ and $R_7$ are not simultaneously hydrogen,

(f) that when $R_1$ and $R_6$ are hydrogen, $R_{13}$ or $R_{14}$ are not simultaneously loweralkyl of 1 to 6 carbon atoms, and

(g) that then wen $R_1$ and $R_9$ taken together form a group of the formula CO, SO, or $CHNR_{11}R_{12}$, $R_6$ and $R_7$ are not simultaneously hydrogen which comprises

a) reacting a compound of the formula 2

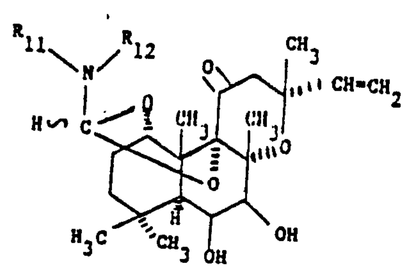

2

wherein $R_{11}$ and $R_{12}$ are as defined above, with 1,1'-carbonyldiimidazole to afford a compound of formula 9

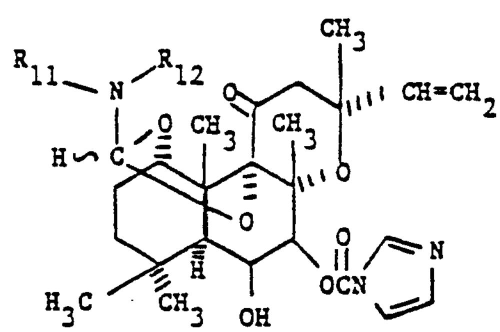

9

treating the same after or without isolation with an amine of the formula $HNR_{13}R_{14}$, wherein $R_{13}$ and $R_{14}$ are as defined above, to afford a compound of the formula 3

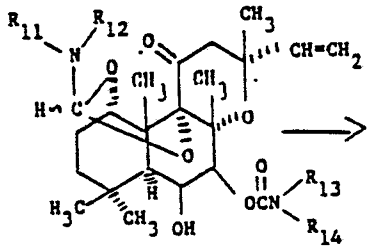

3

and hydrolyzing a compound of the formula 3 with an aqueous alkanol, an aqueous alkanoic acid in alkanol or a mineral acid in aqueous alkanol to afford a compound of the formula 4

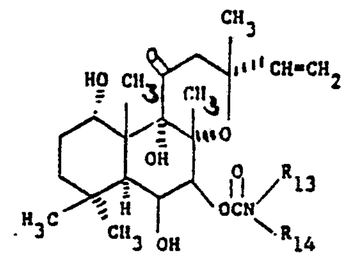

4

or

b) treating a compound of the formula 2 with a compound of the formula 11

$[(R_{17})_3Si]_2NM$ 11

where $R_{17}$ is loweralkyl and M is an alkali metal, to afford an alkali metal alkoxide of the compound of formula 2, and

b₁) treating the compound obtained with either an isocyanate of formula $R_{13}NCO$ or a carbamoyl halide of formula 13

$R_{13}R_{14}N\overset{O}{\overset{\|}{C}}$ Hal 13

where $R_{13}$ and $R_{14}$ are as defined above, to afford a compound of the formula 3, and

$b_2$) hydrolyzing a compound of the formula 3 as described above, to afford a compound of the formula 4,

c) optionally rearranging a compound of the formula 4 by means of an alkali metal alkoxide to afford a compound of the formula 5

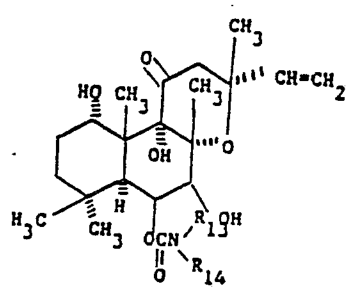

5

wherein $R_{13}$ and $R_{14}$ are as defined above,

d) optionally reacting a compound of the formula 6

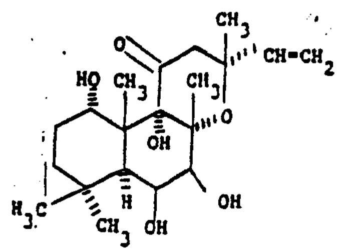

6

with 1,1'-carbonyldiimidazole, followed by the reaction with an amine of the formula $HNR_{13}R_{14}$, where $R_{13}$ and $R_{14}$ are as defined above, according to step a) above to afford a compound of the formula 7

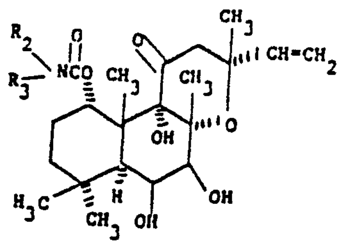

7

wherein $R_2$ and $R_3$ are as defined,

e) optionally reacting a compound of the formula 14

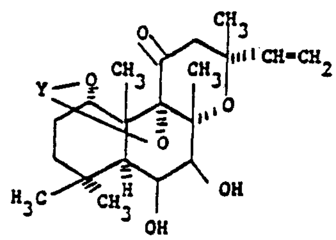

14

wherein Y is SO or CO, with 1,1'-carbonyldiimidazole, followed by the treatment with an amine of the formula $HNR_{13}R_{14}$, wherein $R_{13}$ and $R_{14}$ are as defined above, to afford a compound of the formula 2

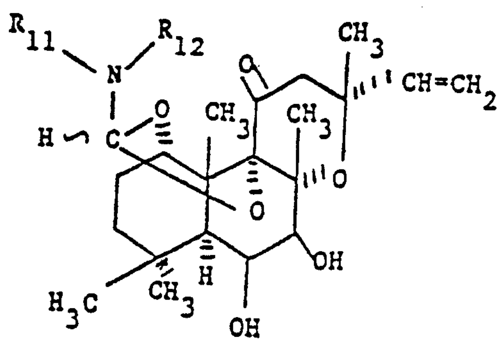

2

or

$e_1$) reacting an alkoxid of a compound of the formula 14 with isocyanate or carbamoyl halide according to step $b_1$) above to afford a compound of the formula 15,

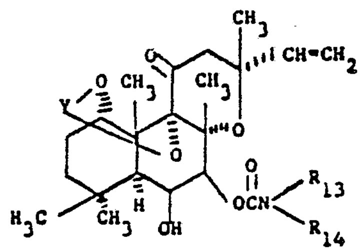

15

and

f) optionally rearranging a compound of the formula 15 according to step c) to afford a compound of the formula 16

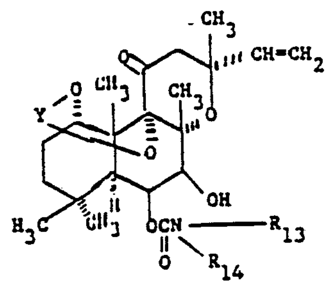

16

wherein $R_{13}$, $R_{14}$ and Y are as defined above.

2. A process as defined in claim 1 wherein $R_1$ is hydrogen and $R_7$ is a group of the formula

$-\overset{\overset{O}{\|}}{C}NR_{14}R_{14}$

where $R_{13}$ and $R_{14}$ are as defined.

3. A process as defined in claim 2 wherein 7β-(aminocarbonyloxy)-8, 13-epoxy-1α, 6β, 9α-trihydroxylabd-14-en-11-one is prepared.

4. A process as defined in claim 2 wherein 7β-(N-methylaminocarbonyloxy)-1α, 6β, 9α-trihydroxylabd-14-en-11-one is prepared.

5. A process as defined in claim 2 wherein, 8,13-epoxy-7β-(2-hydroxethylaminocarbonyloxy) - 1α, 6β-9α-trihydroxylabd-14-en-11-one is prepared.

6. Use of a compound as defined in claim 1 for the preparation of a medicament having intraocular pressure reducing activity.

7. A process for the preparation of a compound of the formula

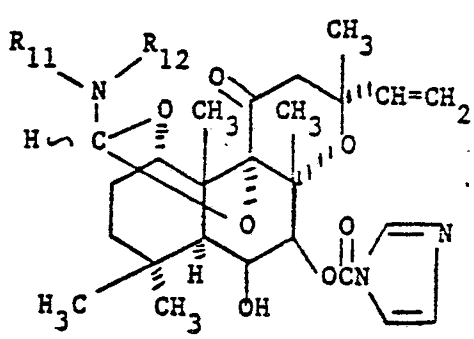

wherein $R_{11}$ and $R_{12}$ are each independently loweralkyl of 1 to 6 carbon atoms; or $R_{11}$ and $R_{12}$ taken together with the nitrogen atom to which they are attached form a group of the formula

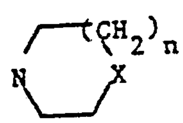

wherein X is O, S or a group of the formula $CHR_4$ wherein $R_4$ is hydrogen, loweralkyl of 1 to 6 carbon atoms, or a group of the formula $OR_5$ wherein $R_5$ is hydrogen, loweralkyl of 1 to 6 carbon atoms or a group of the formula $\overset{O}{\overset{\|}{C}}R_{10}$ wherein $R_{10}$ is loweralkyl of 1 to 6 carbon atoms and n is 0 or 1, which comprises reacting a compound of the formula 14

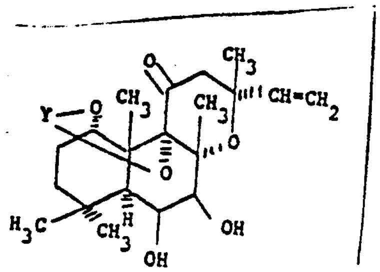

14

where Y is as defined above, with 1,1'-carbonyldiimidazole.

European Patent Office

# EUROPEAN SEARCH REPORT

Application Number

EP 87 11 9241

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 191 166 (HOECHST)<br>* Pages 1,2-5; page 12, example 1; page 13, claims *<br>--- | 1,6 | C 07 D 311/92<br>C 07 D 493/10<br>C 07 D 497/10<br>A 61 K 31/35 |
| A | EP-A-0 193 132 (HOECHST)<br>* Pages 1,2,23,24 *<br>----- | 1,6 | |
| | | | TECHNICAL FIELDS SEARCHED (Int. Cl.4) |
| | | | C 07 D 311/00<br>C 07 D 493/10<br>C 07 D 497/10 |

The present search report has been drawn up for all claims

| Place of search | Date of completion of the search | Examiner |
|---|---|---|
| THE HAGUE | 22-04-1988 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)